# EUROPEAN PATENT APPLICATION

(11) **EP 1 990 080 A1**
(43) Date of publication of application: **12.11.2008**
(21) Application number: 08380136.5
(22) Date of filing: 05.05.2008
(51) Int. Cl.: B01D 53/86, F24F 11/00, A61L 9/20

(54) **Air purifier**

(30) Priority: 07.05.2007 ES 200700934
(71) Applicant: AMBITO DE INVESTIGACION TECNOLOGICA, S.L., 05001 Avila (ES)
(72) Inventor: Martin Velayos, Jose Luis, 05001 Avila (ES); Tapia Del Rey, Felix, 05001 Avila (ES)
(74) Representative: Rodriguez Perez, Jesus

(57) **Abstract**

Air purifier, of the type which incorporate a casing wherethrough is forced to pass an air flow, characterised in that within said casing is established a control module, equipped with a microcontroller, whereto are associated a plurality of sensors as well as a programming display. Said control module incorporates an internal data storage memory and a programming software as well as of an ultraviolet led diode card beside which is established a photocatalytic substance which reacts with the light emitted thereby, purifying the air which passes through the interior of the casing of the device. An infrared module is associated to the control module for control of the device through the complementary remote control. It includes a radio module for data transmission and control thereof through a central computer. An audio stage, where one or more loudspeakers are connected, is associated to said microcontrolle

## Description

### OBJECT OF THE INVENTION

The present invention relates to a device especially devised to purify the air of a room or closed environment by the action of a photocatalytic substance activated by an ultraviolet light.

The object of the invention is to provide an air purifier which permits treating the air in contaminated atmospheres simply and efficiently, making it possible to analyse the characteristics of said air, as well as storing and processing the parameters analysed, with a structure which enables the activation of the device both automatically and manually.

### BACKGROUND OF THE INVENTION

Air quality in indoor environments can be defined as the nature of the air insofar as it affects the health and wellbeing of the occupants of a building or home. The amount of time spent inside these facilities is a reason for concern given the circumstance that the pollution level of the interiors can reach 10 to 100 times higher than the outside concentrations. The symptoms presented by the affected parties are not usually severe and, as they do not cause excessive sick leave, the effects that, however, are translated into a general situation of discomfort, are often minimized. In practice, these effects can alter both the physical and mental health of the worker, causing greater stress and, with it, reduced work performance. To describe these situations, when the symptoms affect more than 20% of the occupants of a building, we speak of the "sick building syndrome".

At present, it is admitted that those atmospheres which do not have natural ventilation and which are closed, to achieve a better performance of the air conditioning system, can be areas of exposure to contaminants. These include offices, public buildings, schools and nurseries, commercial buildings and even private residences. The extent of the damages they may represent for the health is not known accurately, since the initial levels of contaminants that have been determined, mainly in studies carried out in offices and private residences, are usually far below the respective permitted limits of exposure to industrial environments.

On the other hand, the traditional techniques of industrial hygiene are frequently unsuitable and insufficient for finding solutions, since the primary causes of this situation are often difficult to identify.

Although there are numerous types of devices for renewing the air in certain rooms such as those previously mentioned, the applicant has no knowledge of the existence of a device which is capable of evaluating, correcting and and/or notifying of the steps to follow to improve the quality of an environment, and in this way be able to prevent and correct atmospheres hazardous for the health of the occupants of a certain enclosure.

### DESCRIPTION OF THE INVENTION

The air purifier that the invention proposes resolves the different aspects of the aforementioned problem in a fully satisfactory manner.

To do this, the purifier recommended is constituted from a casing, as is conventional, wherein is established a control module, assisted by the corresponding power source, materialized in an electric circuit, equipped with a microcontroller provided with the corresponding programming firmware, which will be mentioned further on, a microcontroller whereto are associated, among other elements, a programming display, a series of sensors, an audio stage, one or more fans, as well as an ultraviolet led card in charge of activating the photocatalytic substance.

More specifically, the purifier will incorporate a series of temperature and moisture sensors, as well as sensors for the concentration of volatile organic compounds and carbon monoxide existing in the air, which enable the microcontroller to evaluate air quality, thanks to its internal firmware, so that the measurements taken thereby are stored in an internal memory associated to said microcontroller.

The purifier can be activated manually, through an infrared remote control, or automatically by its periodic programming or through automatic activation programs in accordance with the parameters detected by the different sensors.

Thus, to purify the air the device will activate the fan or fans associated thereto, according to the aforementioned mode of activation, as well as the ultraviolet led card, beside which will be established an air-purifying photocatalytic substance.

It has also been provided that the device incorporates an audio stage, to activate acoustic signals and alarms by means of the microcontroller when necessary.

In accordance with another of the characteristics of the invention, and although the device is an element perfectly autonomous as regards its operation, it can also be integrated within a wireless network, for which purpose it has a radio module, it being possible to work in different bands and protocols. In this way, the device can be used within large premises, wherein it is intended to improve overall air quality, giving less importance to the particular features of the room where the purifier is found.

The data stored in the internal memory of the device can be consulted through said wireless network, for their interpretation, it being possible to control the actuation thereof by a central computer and/or independently in accordance with the specific parameters of each one.

### DESCRIPTION OF THE DRAWINGS

To complement the description being made and in order to help towards a better understanding of the characteristics of the invention, in accordance with a preferred example of practical embodiment thereof, a set of drawings is attached as an integral part of said description wherein the following has been represented in an illustrative and non-limitative manner:

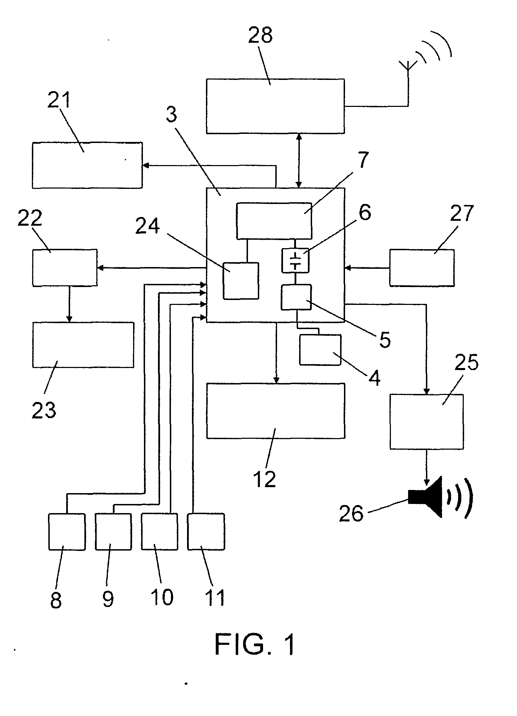

- Figure 1: shows a block diagram corresponding to the internal structure of an air purifier made in accordance with the object of the present invention.
- Figure 2: shows, according to a schematic perspective representation, the external appearance of the purifier of the previous figure.
- Figure 3: shows, according to a front elevational view, a detail of the programming display of the device of the previous figures.

### PREFERRED EMBODIMENT OF THE INVENTION

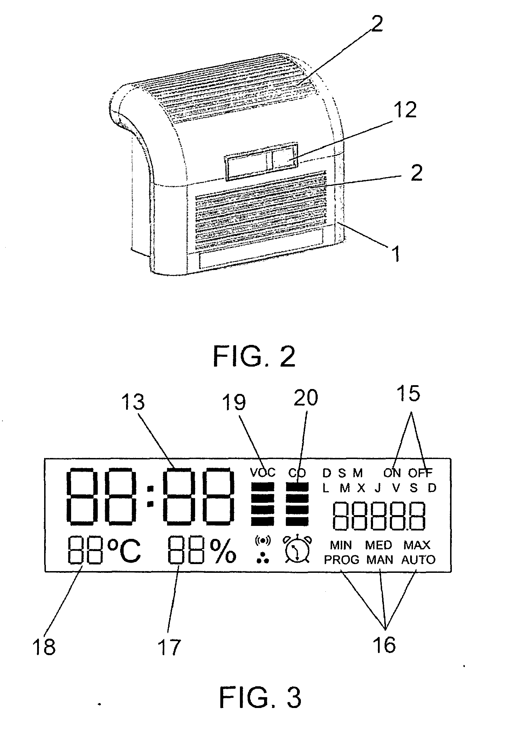

In view of the figures described, it can be observed that the air purifier object of the invention is constituted from a casing (1), which can adopt the configuration shown in figure 2 or any other in accordance with different design lines, without this affecting the essential nature of the invention, a casing (1) provided with grooves (2) for the intake and outlet of air therethrough, and wherein is established a control module (3) materialized in an electronic circuit, supplied by the corresponding power source (4), in charge of transforming the 220 volts from the AC mains current into 12 volt DC current without stabilizing, and at whose outlet is established a voltage regulator and back-up voltage, whereby the DC voltage provided by the power source (4) is adapted to the levels required by the different elements of the device, including a supercondenser (6) which provides power to a microcontroller (7) in a possible cut of electric flow, avoiding the time and date from being lost and the user having to reprogramme them.

In said microcontroller is housed the firmware that governs the behaviour of the device. It handles all the elements that form the device.

To said control device are associated a moisture sensor (8), a temperature sensor (9), a sensor (10) that measures the concentration of volatile organic compounds and a sensor (11) in charge of detecting the carbon monoxide concentration in the air.

Likewise to said control module (3), and therefore to its microcontroller, is associated a display (12), the one represented in detail in figure 3, whereby it is possible to observe the programming data of the device corresponding to the time (13) and date (14), the active or inactive status (15) of the device, the modes of programming (16), as well as the parameters taken by the different sensors, such as moisture (17), temperature (18), concentration of volatile organic compounds (19), concentration of carbon monoxide (20) as well as other parameters corresponding to the maximum, minimum and average values of the different variables measured by the sensors.

The control module also governs the actuation of one or more fans (21), preferably a pair of them, also positioned inside the casing (1), as well as the activation of an ultraviolet led diode card (22), wherein twelve diodes participate, beside which is established a photocatalytic substance (23) which reacts with the light emitted thereby purifying the air. The deposit of said photocatalytic substance (23) will be equipped with a level sensor, not represented in the figures, whereby through the display (12) it is possible to alert the users of the device of the number of hours remaining operative before it is necessary to change said substance.

Thus, the device incorporates a real-time clock function which permits the purifier to suitably perform all the events related with time, also depending on the day of the week in question, enabling its time programming during time frames that the user established in order to provide greater use of energy and the consumable and photocatalytic substance (23).

Likewise and with the object of giving greater convenience to the user, the purifier has an automatic mode wherein the apparatus is capable of deciding, thanks to the data analysed, if the atmosphere of a room needs to be cleaned, automatically acting on the activation of the fan (21) and the ultraviolet led card (22).

The measurement taking of the different sensors (8-9-10-11) will be carried out by the microcontroller (7) with the same periodicity, to give consistency to the database that may be formed from the measurements taken by the equipment, for which purpose it has an internal memory (24) card wherein said data is stored.

From the samples taken by the sensors and of the predefined thresholds, a light indicator will illuminate in the display, that can be accompanied with different acoustic signals, for which reason the control module (3) incorporates an audio stage (25) associated to the corresponding loudspeaker or loudspeakers (26), permitting blind users to notice the circumstance in question.

It has also been provided that the control module incorporates an infrared module (27) so that through a remote control the device can be easily controlled and programmed by the user.

As has previously been commented, the purifier of the invention may constitute an independent and autonomous element, or form part of a purifier system, all connected to a wireless network, for which purpose it will incorporate, in this case, a radio module (28), through which it can be controlled by a central computer, it being possible to transmit the periodic information corresponding to the different parameters measured thereby, so that even forming part of a system it can equally operate autonomously or by its activation through the central computer.

It should finally be mentioned that the purifier will incorporate means of detection of opening of the drawer wherein the purifying substance (23) associated to the control module (3) is established.

## Claims

1. Air purifier, of the type which incorporate a casing wherethrough is forced to pass an air flow, **characterised in that** within said casing is established a control module, supplied by the corresponding power source and equipped with a microcontroller, whereto are associated a plurality of sensors, for temperature, moisture, measurement of the concentration of volatile organic compounds and measurement of the concentration of carbon monoxide in the air, as well as a programming display which reflects the data obtained by said sensors, having provided that said control module incorporates an internal data storage memory and a programming software for the manual or automatic activation of one or more fans positioned within the casing, as well as of an ultraviolet led diode card beside which is established a photocatalytic substance which reacts with the light emitted thereby, purifying the air which passes through the interior of the casing of the device.

2. Air purifier according to claim 1, **characterised in that** an infrared module is associated to the control module for control of the device through the complementary remote control.

3. Air purifier according to claim 1, **characterised in that** it includes a radio module for data transmission and control thereof through a central computer.

4. Air purifier according to claim 1, **characterised in that** an audio stage, whereto one or more loudspeakers are connected, is associated to said microcontroller.
